(19)

Europäisches
Patentamt
European
Patent Office
Office européen
des brevets

(11) **EP 4 597 516 A1**

(12) **EUROPEAN PATENT APPLICATION**

(43) Date of publication:
06.08.2025 Bulletin 2025/32

(21) Application number: 24216826.8

(22) Date of filing: 02.12.2024

(51) International Patent Classification (IPC):
$G16H\ 50/30^{(2018.01)}$      $G16H\ 50/70^{(2018.01)}$

(52) Cooperative Patent Classification (CPC):
G16H 50/70; G16H 50/30

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 30.01.2024 CN 202410132483

(71) Applicant: Beijing Normal University-Hong Kong
Baptist
University United International College
Zhuhai, Guangdong Province 519000 (CN)

(72) Inventors:
• PAN, Jianxin
Zhuhai, Guangdong Province, 519000 (CN)
• WANG, Chunyu
Zhuhai, Guangdong Province, 519000 (CN)
• LUO, Renwen
Zhuhai, Guangdong Province, 519000 (CN)

(74) Representative: Ipside
7-9 Allée Haussmann
33300 Bordeaux Cedex (FR)

(54) **METHOD FOR MODELING BIOMARKER VARIABILITY IN JOINT ANALYSIS OF LONGITUDINAL AND SURVIVAL DATA**

(57) Embodiments of the present invention disclose a joint modeling method, a method for judging the influence of an index on a model and a storage medium, wherein the joint modeling method comprises: determining an expression of a longitudinal index mean function and an expression of a longitudinal index cumulative change rate function; constructing a longitudinal data sub-model and a survival data sub-model; determining a covariance matrix structure of a random error in the longitudinal data sub-model based on a calculation model selection criterion, and calculating a maximum likelihood estimation value of parameters in the longitudinal data sub-model and the survival data sub-model by adopting an EM algorithm, and predicting a multidimensional random effect in the longitudinal index mean function to obtain the estimation of a smooth function in the longitudinal index mean function.

| |
|---|
| Determining an expression of a longitudinal index mean function and an expression of a longitudinal index cumulative change rate function   S101 |
| ↓ |
| Constructing a longitudinal data sub-model, wherein longitudinal observation data in the longitudinal data sub-model at least depends on the longitudinal index mean value and a random error   S102 |
| ↓ |
| Constructing a survival data sub-model, wherein a survival risk function in the survival data sub-model at least depends on the longitudinal index mean value and the longitudinal index cumulative change rate   S103 |
| ↓ |
| Determining a covariance matrix structure of the random error based on a calculation model selection criterion   S104 |
| ↓ |
| Calculating a maximum likelihood estimation value of parameters in the longitudinal data sub-model and the survival data sub-model by adopting an EM algorithm, and predicting the multidimensional random effect to obtain the estimation of the smooth function   S105 |

FIG. 1

EP 4 597 516 A1

**Description**

TECHNICAL FIELD

The present invention relates to the technical field of data analysis, and in particular, relates to a joint modeling method, a method for judging the influence of an index on a model and a storage medium.

BACKGROUND

Cardiovascular disease is one of the most deadly diseases in the world. According to the report of the World Health Organization, the number of people who die of heart disease in the world is as high as 17.9 million every year, among which nearly 80% die of heart attack or stroke, and nearly 35% are under 70 years old. Traditionally, it is believed that the risk probability of disease attack can be monitored through regular observation of patients' blood pressure, blood sugar and other factors. In 2010, Professor Rothwell's team from Oxford University consecutively published three articles in the top medical journal The Lancet, pointing out that it is very limited to only observe the level of blood pressure, blood sugar and other factors. Patients with normal average blood pressure but high rate of change may be at a higher risk of stroke or death than those with high but relatively stable blood pressure. This conclusion has caused great concern and controversy in the cardiovascular academic field. If the conclusion holds, it may change people's traditional cognition of cardiovascular diseases. However, this conclusion is drawn from the research of medical mechanism, and there is not a lot of statistical data analysis to support this view currently. At present, no statistical model, theory and algorithm is available for analyzing the influence of blood pressure change rate on the risk of stroke or death, and analyzing how to compare the two factors of blood pressure level and blood pressure change rate, which is more important for risk prediction.

Although the above problems originated from medicine, they are also common in other fields. For example, in financial technology, people tend to pay more attention to the rate of change of stock price than the correlation between the stock price itself and a certain risk event. For another example, in industrial manufacturing, people tend to pay more attention to the influence of pressure change on product life in the manufacturing process of a certain product, and so on. All these problems can be categorized as the problem of survival data modeling with independent variables of longitudinal index change rate.

When the longitudinal index process changes and it is necessary to judge the influence of the longitudinal index change rate on the statistical inference of the risk model of survival data, the commonly used techniques in the prior art include: a) when the longitudinal index process has change points, judging the problem for which the longitudinal index has change points by parametric and nonparametric algorithms when the position and number of the change points are unknown; b) simply regarding the repeated observation value of each individual, that is, the sample standard deviation, coefficient of variation or continuous coefficient of variation of longitudinal data as the independent variable of the survival data model; c) taking the mean function of the longitudinal index at the current time t or the first derivative of the function at the time t as the independent variable of the survival data model; and d) taking the variance or logarithm of the variance of the random error of the longitudinal data as a random effect and adding it into the survival data model in the form of a vulnerability factor for analysis.

The above technique a) can depict the local change of the longitudinal index, but cannot judge the global dynamic change rate of the longitudinal index. The technique b) is a common method used by medical researchers which features simple and easy operation; however, these measures based on samples are not sufficient and stable in describing the change rate, and especially when the number of repeated observations is limited, serious deviation will be caused to the parameter estimation of the survival data model, which leads to wrong analysis results. The algorithm of the technique c) takes into account the systematic influence of the longitudinal index on the survival model, but it only uses the value of the longitudinal index and its first derivative at the current time point t without considering the cumulative change rate of the longitudinal index until the time t. The algorithm of the technique d) introduces the measurement error variance of longitudinal data into the survival model, or regards the logarithm of the error variance as a random effect (i.e., a vulnerability factor) in the survival model; however, this algorithm cannot systematically describe the cumulative change rate of the longitudinal index and lacks theoretical basis. Therefore, an urgent need exists in the art to provide a statistical algorithm that can not only describe the longitudinal index cumulative change rate systematically and dynamically, but also compare the importance of influence by the longitudinal index mean value and the longitudinal index cumulative change rate on the survival risk in the survival model.

SUMMARY

Embodiments of the present invention aim to provide a joint modeling method, a method for judging the influence of an index on a model and a storage medium, so as to solve the problems in the prior art that the survival data model cannot systematically describe the longitudinal index change rate and the influence of the longitudinal index cumulative change rate on the survival data model is not judged.

In order to solve the above technical problems, embodiments of the present invention provide the following technical solutions.

According to an aspect of the present invention, there is provided a joint modeling method based on longitudinal data and survival data, and the method comprises:

determining an expression of a longitudinal index mean function and an expression of a longitudinal index cumulative change rate function, wherein a longitudinal index mean value in the longitudinal index mean function depends on a first reference independent variable in a parameter form and depends on a smooth function based on a multidimensional random effect in a time form, and a longitudinal index cumulative change rate in the longitudinal index cumulative change rate function is described as a power root of a varicose measure of the longitudinal index mean function;

constructing a longitudinal data sub-model, wherein longitudinal observation data in the longitudinal data sub-model at least depends on the longitudinal index mean value and a random error;

constructing a survival data sub-model, wherein a survival risk function in the survival data sub-model at least depends on the longitudinal index mean value and the longitudinal index cumulative change rate;

determining a covariance matrix structure of the random error based on a calculation model selection criterion; and calculating a maximum likelihood estimation value of parameters in the longitudinal data sub-model and the survival data sub-model by adopting an EM algorithm, and predicting the multidimensional random effect to obtain the estimation of the smooth function.

Optionally, the expression of the longitudinal index cumulative change rate function is:

$$I_i(t) = \left[ \int_{t_0}^{t} \{m_i''(s)\}^2 \, ds \right]^{1/2}$$

or

$$I_i(t) = \left[ \int_{t_0}^{t} \omega_{\sigma_2}(t - s)\{m_i''(s)\}^2 \, ds \right]^{1/2}$$

wherein $m_i(s)$ is the longitudinal index mean function of the ith individual at time s, $m_i''(s)$ is the second derivative of $m_i(s)$, $I_i(t)$ represents the longitudinal index cumulative change rate of $m_i(t)$ over a time period $[t_0, t]$, $\omega_{\sigma_2}(t - s)$ is a weight function about the parameter $\sigma_2$, and the smaller the distance between s and t is, the greater the weight of $\omega_{\sigma_2}(t - s)$ will be.

Optionally, the expression of the longitudinal index mean function is:

$$\begin{cases} m_i(t) = x_i^T \beta + f_i(t) \\ f_i(t) = \sum_{k=1}^{q} (\xi_k + b_{ik}) B_k(t) \end{cases}$$

wherein xT is a first reference independent variable vector of the ith individual, $\beta$ is a regression coefficient corresponding to $x_i^T$, $f_i(t)$ is the smooth function of the ith individual, $q$ represents the dimension of the random effect, $\xi_k$ is a fixed effect parameter of a $k$th dimension, $b_{ik}$ represents a random effect parameter of the $k$th dimension of the ith individual, and $B_k(t)$ represents a B-spline basis function corresponding to the $k$th dimension.

Optionally, the longitudinal data sub-model is:

$$Y_{ij} = m_i(t_{ij}) + w_{ij}^T \eta + \epsilon_{ij}$$

wherein $Y_{ij}$ is an observation value of a random variable $Y$ at a jth time point $t_{ij}$ for the ith individual, $j = 1, 2, ..., n_i$, $n_i$ represents the total number of repeated observations of $Y$ before a survival time $T_i$ for the ith individual, $w_{ij}$ is an external independent variable corresponding to the ith individual at the jth time point, $\eta$ is an unknown parameter corresponding to $w_{ij}$, and $\varepsilon_{ij}$ is the random error of the ith individual at the jth time point.

Optionally, the survival data sub-model is:

$$\lambda_i(t) = \lambda_0(t) exp\left( z_i^T \gamma + \alpha_1 m_i(t) + \alpha_2 \left[ \int_{t_0}^t \{m_i''(s)\}^2 \, ds \right]^{1/2} \right)$$

or

$$\lambda_i(t) = \lambda_0(t) exp\left\{ z_i^T \gamma + \alpha_1 \int_{t_0}^t \omega_{\sigma_1}(t-s) m_i(s) ds + \alpha_2 \left[ \int_{t_0}^t \omega_{\sigma_2}(t-s) \{m_i''(s)\}^2 \, ds \right]^{1/2} \right\}$$

wherein $\lambda_0(t)$ is a reference risk function, $z_i^T$ is a second reference independent variable vector of the ith individual, $\gamma$ is a co-parameter corresponding to $z_i^T$, $\alpha_1$ is a co-parameter of the longitudinal index mean value $m_i(t)$ at a current time point t, $\alpha_2$ is a co-parameter of the cumulative change rate of $m_i(t)$ before the current time point t; $\omega_{\sigma 1}(t-s)$ is a weight function about the parameter $\sigma_1$, and $\omega_{\sigma 2}(t-s)$ is a weight function about the parameter $\sigma_2$, so that the smaller the distance between s and $t$ is, the greater the weight of the $\omega_{\sigma 1}(t-s)$ and $\omega_{\sigma 2}(t-s)$ will be.

Optionally, the method comprises:
selecting one of two survival data sub-models as the survival data sub-model for data analysis based on the calculation model selection criterion.

Optionally, the random error obeys a multivariate normal distribution with a mean value of 0 and a covariance matrix of $\Sigma_i$, and the covariance matrix $\Sigma_i$ is modeled based on the following equation:

$$T_i \Sigma_i T_i^T = D_i$$

wherein $T_i$ is a lower triangular matrix with a diagonal element of 1, while $D_i$ is a diagonal matrix with a positive diagonal element, the lower triangular element of $T_i$ is $-\phi_{ijk}(1 \le k < j \le n_i)$, the diagonal element of $D_i$ is $\sigma_{ij}^2(1 \le j \le n_i)$, $\phi_{ijk}$ is an autoregressive coefficient of $Y_{ij}$ to $Y_{ik}(k = 1,2, ...j - 1)$, and $\sigma_{ij}^2$ is an innovation variance.

Optionally, the method further comprises:

modeling the $\phi_{ijk}$ and the $\sigma_{ij}^2$ based on the following equation:

$$\begin{cases} \phi_{ijk} = a_{ijk}^T \tau \\ log\left(\sigma_{ij}^2\right) = c_{ij}^T \zeta \end{cases}$$

wherein $a_{ijk}$ and $c_{ij}$ are known independent variables, and $\tau$ and $\zeta$ are model parameters.

According to another aspect of the present invention, there is provided a method for judging the influence of an index on a model, and the method comprises:

obtaining a survival data sub-model by adopting the joint modeling method based on longitudinal data and survival data according to any of the above description;
assuming that the regression coefficient corresponding to the longitudinal index cumulative change rate in the survival data sub-model is 0, determining whether the longitudinal index cumulative change rate influences the survival risk function based on a likelihood ratio criterion or Z-test statistics; and/or,
assuming that the regression coefficient corresponding to the longitudinal index cumulative change rate in the survival data sub-model is greater than a regression coefficient corresponding to the longitudinal index mean value, determining whether the influence of the longitudinal index cumulative change rate on the survival risk function is greater than the influence of the longitudinal index mean value on the survival risk function based on the likelihood ratio criterion or Z-test statistics.

According to yet another aspect of the present invention, there is provided a computer-readable storage medium, wherein the computer-readable storage medium stores a computer program which, when being executed by a processor, causes the processor to perform the steps of the method according to any of the above description.

Beneficial effects of the embodiments according to the present invention are as follows: unlike the situation in the prior art, the embodiment of the present invention provides a joint modeling method based on longitudinal data and survival data, in which first an expression of a longitudinal index mean function and an expression of a longitudinal index cumulative change rate function are determined; then a longitudinal data sub-model and a survival data sub-model are constructed respectively based on the longitudinal index mean function and the longitudinal index cumulative change rate function; and finally, a covariance matrix structure of a random error in the longitudinal data sub-model is determined based on a calculation model selection criterion, and a maximum likelihood estimation value of parameters in the longitudinal data sub-model and the survival data sub-model is calculated by adopting an EM algorithm, and a multidimensional random effect in the longitudinal index mean function is predicted to obtain the estimation of a smooth function in the longitudinal index mean function. The method of the present invention can not only describe the longitudinal index cumulative change rate systematically and quantitatively in the survival data model, but also judge the influence of the longitudinal index cumulative change rate on the survival data risk model and compare the influence of the longitudinal index mean value and the longitudinal index change rate on the survival data model.

BRIEF DESCRIPTION OF THE DRAWINGS

One or more embodiments are exemplarily described with reference to pictures in corresponding attached drawings, and these exemplary descriptions are not intended to limit the embodiments. In the attached drawings, elements with the same reference numerals represent the similar elements, and unless otherwise stated, the pictures in the attached drawings are not intended to limit the scale.

FIG. 1 is a schematic flowchart diagram of a joint modeling method based on longitudinal data and survival data according to an embodiment of the present invention.
FIG. 2 is a schematic view illustrating blood pressure value fitting trajectory curves and cumulative change rate curves of blood pressure values within an observation period of six random patients according to an embodiment of the invention.
FIG. 3 is a schematic flowchart diagram of a method for judging the influence of an index on a model according to an embodiment of the present invention.

DETAILED DESCRIPTION

To make the objectives, technical solutions and advantages of embodiments of the present invention more apparent, the technical solutions of the embodiments of the present invention will be described clearly and completely with reference to the attached drawings illustrating the embodiments of the present invention. Obviously, the embodiments described herein are only a part of but not all of the embodiments of the present invention. All other embodiments that can be obtained by those of ordinary skill in the art from the embodiments of the present invention without making creative efforts shall fall within the scope of the present invention.

In addition, technical features involved in various embodiments of the present invention described below can be combined with each other as long as they do not conflict with each other.

It shall be noted that, the steps shown in the flowchart diagrams of the accompanying drawings may be executed in a computer system such as a set of computer-executable instructions, and although a logical order is shown in the flowchart diagram, in some cases, the steps shown or described may be executed in an order different from that shown herein.

For the convenience of expression, some mathematical symbols appearing in this patent are introduced as follows: suppose there are $N$ individuals in total, and $T_i^*$ and $C_i$ respectively represent the occurrence time and censoring time of a target event of the ith individual ($i = 1, 2, ..., N$). Because of censoring, the data that can be actually observed is

$$T_i = min\{T_i^*, C_i\}$$

and $\delta_i = I\{T_i \leq C_i\}$, wherein $I\{\cdot\}$ is a characteristic function. It is assumed that longitudinal data of a key index observed at the ith individual is $Y_i = (Y_{i1}, ..., Y_{ini})^T$, wherein $Y_{ij}$ is the observation value of a random variable $Y$ at the $j$th time point $t_{ij}(t_{ij} \leq T_i)$ for the $i$th individual ($j = 1, 2, ..., n_i$), $n_i$ represents the total number of repeated observations of $Y$ before the survival time $T_i$ for the ith individual. Obviously, there will be errors in the observation of $Y$ at any time point, and the real but unknown observation value thereof is described as $m_i(t)$ (i.e., the longitudinal index mean value described later). It is assumed that $m_i(t)$ is a smooth function of $t$, and it is assumed that the censoring event and the event occurrence time $T_i^*$ are independent of each other.

Embodiment 1

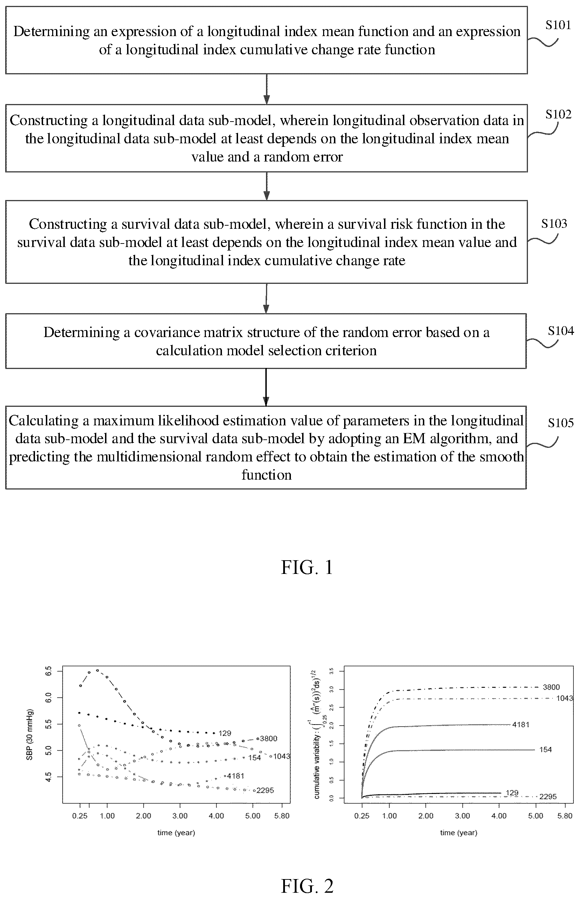

Referring to FIG. 1, there is shown a flowchart diagram of a joint modeling method based on longitudinal data and survival data provided according to an embodiment of the present invention, and the method comprises:

Step S101: determining an expression of a longitudinal index mean function and an expression of a longitudinal index cumulative change rate function.

In some embodiments, a longitudinal index mean value depends on a reference independent variable in a parameter form and depends on a smooth function based on a multidimensional random effect in a time form. Specifically, the longitudinal index mean function is the following parameter regression model:

$$m_i(t) = x_i^T \beta + f_i(t) \qquad (1)$$

wherein xT is a first reference independent variable vector of the ith individual (such as the age and gender or the like of the ith individual), $\beta$ is a regression coefficient corresponding to $x_i^T$, and $f_i(t)$ is the smooth function of the ith individual, which reflects the trajectory of the longitudinal data in time, and different individuals have different trajectories.

In some embodiments, $f_i(t)$ is estimated by using the B-spline regression of multidimensional random effects, specifically as follows:

$$f_i(t) = \sum_{k=1}^{q} (\xi_k + b_{ik}) B_k(t) \qquad (2)$$

wherein $q$ represents the dimension of the random effect, $\xi_k$ is a fixed effect parameter of the $k$th dimension, $b_{ik}$ represents the random effect parameter of the $k$th dimension of the ith individual, and $B_k(t)$ represents the B-spline basis function corresponding to the kth dimension. Specifically, $\{B_k(\cdot)\}_{k=1}^{q}$ is a $q$ dimensional cubic B-spline basis function with a series of fixed nodes.

In some embodiments, the $q$ dimensional fixed effect parameter is expressed as $\xi = (\xi_1, ..., \xi_q)^T$, and the $q$ dimensional random effect parameter of the $i$th individual is expressed as $b_i = (b_{i1}, ..., b_{iq})^T$. Usually, it is assumed that $b_i \sim N_q(0, D)$, i.e., $b_i$ obeys a multivariate normal distribution with a mean value of 0 and a covariance matrix of $D$. $\xi$ and $D$ are unknown parameters.

In some embodiments, the cumulative change rate of $m_i(t)$ is measured with a power root of a varicose measure of the longitudinal index mean function, and specifically, the expression of the longitudinal index cumulative change rate function is:

$$I_i(t) = \left[ \int_{t_0}^{t} \{m_i''(s)\}^2 \, ds \right]^{1/2} \qquad (3)$$

wherein $m_i(s)$ is the longitudinal index mean function of the ith individual at time s, $m_i''(s)$ is the second derivative of $m_i$ $(s)$, and $I_i(t)$ represents the longitudinal index cumulative change rate of $m_i(t)$ over a time period $[t_0, t]$. The reason why the power root is taken instead of the integral itself is that when discussing the impact on survival risk, the cumulative change rate measurement and the mean value $m_i(t)$ should be calibrated on the same scale. Obviously, $I_i(t)$ changes with time t, so it reflects the dynamic nature of the cumulative change rate over $[t_0, t]$. $I_i(t)$ may be introduced into a Cox risk proportion model of survival data as an independent variable, just like the longitudinal index mean function $m_i(t)$, so that the corresponding regression coefficient can be used to judge the influence of the longitudinal index mean value $m_i(t)$ and the longitudinal index cumulative change rate $I_i(t)$ on the survival risk function $\lambda_i(t)$ at the current time $t$, and can also be used to compare the influence of $m_i(t)$ and $I_i(t)$ respectively on $\lambda_i(t)$, which will be detailed later.

FIG. 2 gives an intuitive example of judging the change rate of smooth function curves by the power root $I_i(t)$ of varicosity measurement. The above data comes from a clinical trial of cardiovascular disease in the elderly aged 65-74 by the British Medical Research Council. The graph on the left of FIG. 2 shows the fitting trajectory curves of blood pressure values (systolic blood pressure) of six randomly selected patients, and for the convenience of statistics, the blood pressure values are divided by 30. The graph on the right of FIG. 2 shows the curve of cumulative change rate $I_i(t)$ within the

observation period. It can be seen that patients with mild blood pressure levels, such as patients No. 129 and No. 2295, have a small cumulative change rate; and on the contrary, patients with large changes in blood pressure levels, such as patients No. 3800 and No. 106, have a relatively large cumulative change rate $I_i(t)$ in blood pressure. In addition, $I_i(t)$ also reveals where the cumulative change rate of $m_i(t)$ is relatively large and where it begins to level off. Therefore, Equation (3) is a reasonable measure that can systematically and dynamically judge the cumulative change rate of the smooth function $m_i(t)$.

Step S102: constructing a longitudinal data sub-model, wherein longitudinal observation data in the longitudinal data sub-model depends on the longitudinal index mean value and a random error.

In some practical problems, the observation value $Y_{ij} = Y_i(t_{ij})$ is not only influenced by an internal reference independent variable $x_i$, but may also be interfered by external factors or independent variables. For example, in blood pressure measuring of patients, the results measured by doctors or nurses are often different, because psychological fear of doctors often leads to a sharp increase in blood pressure for some patients, which is medically called a "white coat phenomenon". Furthermore, the blood pressure values of the elderly vary in different seasons. Therefore, the following longitudinal data models are considered in the present invention:

$$Y_{ij} = m_i\left(t_{ij}\right) + w_{ij}^T \eta + \epsilon_{ij} \qquad (4)$$

wherein $Y_{ij}$ is an observation value of a random variable $Y$ at a jth time point $t_{ij}$ for the ith individual, $j = 1, 2, ..., n_i$, $n_i$ represents the total number of repeated observations of $Y$ before a survival time $T_i$ for the ith individual, $w_{ij}^T$ is an external independent variable vector corresponding to the ith individual at the jth time point, $\eta$ is a parameter corresponding to $w_{ij}^T$, and $\varepsilon_{ij}$ is the random error at the jth time point of the ith individual.

The random error of the ith individual at each measurement is expressed as $\varepsilon_i = (\varepsilon_{i1}, ..., \varepsilon_{ini})^T$, and it is assumed that $\varepsilon_i \sim N_{ni}(0, \Sigma_i)$, i.e., $\varepsilon_i$ obeys a multivariate normal distribution with a mean value of 0 and a covariance matrix of $\Sigma_i$. Here $\Sigma_i$ is an unknown $n_i \times n_i$ dimensional positive definite covariance matrix.

The covariance structures commonly used in longitudinal data analysis are independent covariance structures, uniform variance structures and first-order autoregressive AR(1) structure or the like. These parameterized covariance structures feature a simple structure, and may transform the estimation of the matrix $\Sigma_1, ..., \Sigma_N$ into the estimation of a few parameters. However, once the assumption of these parameter structures is wrongly set, deviation or even serious errors will be caused to the parameter estimation of the survival data model. Therefore, the present invention proposes to model $\Sigma_i$ as well so that which kind of structure that $\Sigma_i$ belongs to and influence of $\Sigma_i$ on the statistical inference of the survival data model are determined by data.

Specifically, based on the Minimum Covariance Determinant (MCD) model, the covariance matrix $\Sigma_i$ is modeled according to the following equation:

$$T_i \Sigma_i T_i^T = D_i \qquad (5)$$

wherein $T_i$ is a lower triangular matrix with a diagonal element of 1, while $D_i$ is a diagonal matrix with a positive diagonal element, the lower triangular element of $T_i$ is $-\phi_{ijk}(1 \le k < j \le n_i)$, the diagonal element of $D_i$ is $\sigma_{ij}^2 (1 \le j \le n_i)$, $\phi_{ijk}$ is an autoregressive coefficient of $Y_{ij}$ to $Y_{ik}(k = 1, 2, ...j - 1)$, and $\sigma_{ij}^2$ is an innovation variance.

Further speaking, $\phi_{ijk}$ and $\sigma_{ij}^2$ are modeled based on the following equation:

$$\begin{cases} \phi_{ijk} = a_{ijk}^T \tau \\ log\left(\sigma_{ij}^2\right) = c_{ij}^T \zeta \end{cases} \qquad (6)$$

wherein $a_{ijk}$ and $c_{ij}$ are known independent variables, and $\tau$ and $\zeta$ are model parameters.

In the embodiment of the invention, a covariance matrix modeling method between individual repeated observation values in longitudinal data is proposed, so that the covariance structure fits with the data to avoid the problem in the prior art that the parameter estimation of the survival data model will be negatively affected greatly (such as deviations occur to parameter estimation) if the assumption is wrongly set when a linear mixed model or a generalized linear model is adopted (usually it is assumed that observation errors are identically distributed independently from each other). This method is a data-driven method, and the covariance structure of its random error can be given through the longitudinal data itself.

To sum up, in the longitudinal data sub-model (4), the known information is $Y_{ij}$, $x_i$ and $w_{ij}$, the unknown parameter is $\beta$, $\eta$ and $\Sigma_i$, and the unknown function is $fi(t)$.

Step S103: constructing a survival data sub-model, wherein a survival risk function in the survival data sub-model depends on the longitudinal index mean value and the longitudinal index cumulative change rate.

It is assumed that the risk function of the occurrence time $T_i^*$ of a target event depends on both the longitudinal index mean value $m_i(t)$ and the cumulative change rate of $m_i(t)$ before the time t, and the following generalized Cox risk model of survival data is considered:

$$\lambda_i(t) = \lambda_0(t)exp\left(z_i^T\gamma + \alpha_1 m_i(t) + \alpha_2\left[\int_{t_0}^t \{m_i''(s)\}^2\, ds\right]^{1/2}\right) \quad (7)$$

wherein $\lambda_0(t)$ is a reference risk function, $z_i^T$ is a second reference independent variable vector of the ith individual, $\gamma$ is a co-parameter corresponding to $z_i^T$, $\alpha_1$ is a co-parameter of a longitudinal index mean value $m_i(t)$ at a current time point t, and $\alpha_2$ is a co-parameter of the cumulative change rate of $m_i(t)$ before the current time point t.

In addition, the calculation equation of the risk rate of occurrence of the target event at the time t is:

$$\lambda_i(t) = \lim_{\Delta t \to 0} \frac{1}{\Delta t} P\{t \le T_i^* < T + \Delta t | T_i^* \ge t\} \quad (8)$$

In the survival data sub-model (7), the survival risk function $\lambda_i(t)$ only depends on the longitudinal index mean value $m_i(t)$ at the current time point $t$ and the cumulative change rate of $m_i(t)$ before the time $t$. In many practical problems, it may not be reasonable to only consider the longitudinal index mean value $m_i(t)$ at the current time point t. For example, in medical research on heart diseases, we need to consider not only the blood pressure value at the current time point, but also the average value of blood pressure in the past period of time as well as the rate of change of blood pressure within this period. For another example, the evaluation of the curative effect of a medicament often refers to the influence of medicament administration in the past period of time on the current health status. Naturally, the historical value $m_i(s)(s < t)$ far away from the current time point $t$ and its cumulative change rate has little influence on the survival risk function $\lambda_i(t)$; and on the contrary, $m_i(s)(s < t)$ close to the current time point $t$ and its cumulative change rate has great influence on $\lambda_i(t)$. In order to reflect this phenomenon in the model, the following risk model is proposed, which weights the historical longitudinal index and its cumulative change rate:

$$\lambda_i(t) = \lambda_0(t)exp\left\{z_i^T\gamma + \alpha_1\int_{t_0}^t \omega_{\sigma_1}(t-s)m_i(s)ds + \alpha_2\left[\int_{t_0}^t \omega_{\sigma_2}(t-\right.\right.$$

$$\left.\left. s)\{m_i''(s)\}^2\, ds\right]^{1/2}\right\} \quad (9)$$

In the survival data sub-model (9), $\omega_{\sigma_1}(t-s)$ is a weight function about the parameter $\sigma_1$, and $\omega_{\sigma_2}(t-s)$ is a weight function about the parameter $\sigma_2$, wherein the smaller the distance between s and $t$ is, the greater the weight of the $\omega_{\sigma_1}(t-s)$ and $\omega_{\sigma_2}(t-s)$ will be. The integral term $\left[\int_{t_0}^t \{m_i''(s)\}^2\, ds\right]$ describing the cumulative change rate of $m_i(t)$ in equation (3) is transformed into $\left[\int_{t_0}^t \omega_{\sigma_2}(t- s)\{m_i''(s)\}^2\, ds\right]$.

In some embodiments, the longitudinal index include blood pressure index and/or blood glucose index, the first reference independent variable vector and the second reference independent variable vector include an individual's age and gender, and the external independent variable is an external factor variable that affects the blood pressure index or the blood glucose index.

In some embodiments, suppose $u = t - s$, and the functional forms of $\omega_{\sigma_1}(t-s)$ and $\omega_{\sigma_2}(t-s)$ may be taken as:

$$\omega_\sigma(u) = \omega_\sigma(u; [0, t - t_0]) = \frac{(1/\sigma)\phi(u/\sigma)}{\Phi((t-t_0)/\sigma) - \Phi(0)} \qquad (t > t_0) \quad (10)$$

wherein $\phi(\cdot)$ and $\Phi(\cdot)$ are respectively the density function and cumulative distribution function of standard normal distribution $N(0,1)$, and the denominator term plays a regularization role so that $\int_0^{t-t_0} \omega_\sigma(u) du = 1$. In fact, $\omega_\sigma(u; [0, t-t_0])$ is a density function of truncated normal distribution with a mean value of 0 and a variance of $\sigma^2$ over the interval $[0, t-t_0]$. When s is close to t, a larger weight is given to $\omega_\sigma(t-s)$, and when s is far away from t, a smaller weight is given to $\omega_\sigma(t-s)$, thus reflecting the fact that the longitudinal index mean value $m_i(s)$ and the longitudinal index cumulative change rate closer to the time t may have a greater influence on the survival risk function at the time t, while those far away from the time t may have a smaller influence thereon. The parameter $\sigma$ controls the descending speed of the weight $\omega_\sigma(u)$ (u > 0), and when $\sigma$ is large, the weight $\omega_\sigma(u)$ decreases gently with the increase of time distance u, so the model (9) can cover the longitudinal data information and change rate information over a long time in the past, and can reflect how the time distance affects the influence on $\lambda_i(t)$. Particularly, when $\sigma$ is very large, $\omega_\sigma(u)$ approximate to the equal weight function 1/u, while when $\sigma$ is very small, $\omega_\sigma(u)$ may only include the information of the longitudinal index at the current time t, that is, it has little to do with historical information. In the model (9), $\sigma_1$ and $\sigma_2$ are both unknown parameters, which need to be estimated based on longitudinal data and survival data. Therefore, the model (9) provides a more flexible survival risk model that can truly reflect the longitudinal historical information.

After approximation of $f_i(t)$ with B-spline, the integral terms $\left[ \int_{t_0}^{t} \{m_i''(s)\}^2 ds \right]$ and $\left[ \int_{t_0}^{t} \omega_{\sigma_2}(t-s)\{m_i''(s)\}^2 ds \right]$ describing the cumulative change rate of $m_i(t)$ in models (7) and (9) can be expressed as a certain quadratic form of $\tilde{b}_i = \xi + b_i$, that is, $\left[ \int_{t_0}^{t} \{m_i''(s) ds \right]^{1/2}$ or $\left[ \int_{t_0}^{t} \omega_{\sigma_2}(t-s)\{m_i''(s)\}^2 ds \right]$ equals to $\tilde{b}_i^T K(t_0, t) \tilde{b}_i$, wherein $K(t_0, t)$ is a matrix that only depends on the sample basis function $\{B_k(\cdot)\}_{k=1}^{q}$. At this time, the models (7) or (9) is transformed into

$$\lambda_i(t) = \lambda_0(t) \exp\left[ z_i^T \gamma + \alpha_1\{x_i^T \beta + \tilde{b}_i^T B(t)\} + \alpha_2\{\tilde{b}_i^T K(t_0, t)\tilde{b}_i\}^{1/2} \right] \quad (11)$$

wherein $B(t) = (B_1(t), ..., B_q(t))^T$, that is, the estimation problem with nonparametric regression function and cumulative change rate in the model (7) or (9) is transformed into the parameter estimation problem in the model (11).

Step S104: determining a covariance matrix structure of the random error based on a calculation model selection criterion.

When the covariance matrix is modeled by the MCD model, the covariance matrix $\Sigma_i$ can be estimated directly, but the dimension of covariates $a_{ijk}$ and $c_{ij}$ in the model needs to be determined based on the calculation model selection criterion. Generally, the covariates $a_{ijk}$ and $c_{ij}$ may be set as a polynomial of time, and the order of polynomial is related to the dimension of covariates $a_{ijk}$ and $c_{ij}$. Once the dimension of the covariates $a_{ijk}$ and $c_{ij}$ is determined, the polynomial of the covariates $a_{ijk}$ and $c_{ij}$ is determined, and then the covariance matrix structure of the random error is determined.

In some embodiments, the calculation model selection criterion is the AIC criterion (also called Akaike information criterion) or the BIC criterion (also called Bayesian information criterion).

Similarly, it is also used to select one of the model (7) and the model (9) as the survival data sub-model for data analysis based on the above calculation model selection criteria.

Step S105: calculating a maximum likelihood estimation value of parameters in the longitudinal data sub-model and the survival data sub-model by adopting an EM algorithm, and predicting the multidimensional random effect to obtain the estimation of the smooth function.

The maximum likelihood estimation of parameters is obtained by the Expectation-Maximum (EM) algorithm, wherein the parameters include the regression coefficient $\beta$ of the first reference independent variable vector $x_i^T$, parameter $\eta$ corresponding to an external independent variable $w_{ij}^T$, covariance matrix $\Sigma_i$ corresponding to the random error $\varepsilon_i$, co-parameter $\gamma$ corresponding to the second reference independent variable vector $z_i^T$, co-parameter $\alpha_1$ of the longitudinal index mean value $m_i(t)$ at the current time point t, co-parameter $\alpha_2$ of the cumulative change rate of $m_i(t)$ before

the current time point t, $q$ dimensional fixed effect parameter $\xi$, and the covariance matrix $D$ corresponding to the $q$ dimensional random effect parameter $b_i$. The random effect parameter $b_i$ and the random error $\varepsilon_i$ are independent of each other, and the first reference independent variable vector $x_i^T$ and the second reference independent variable vector $z_i^T$ may be the same as or different from each other. Because B-spline and EM algorithm are guaranteed in theory and are also commonly used function approximation and optimization methods in statistics, so this is feasible both in terms of theory and technology.

In some embodiments, the Best Linear Unbiased Prediction (BLUP) is made for multidimensional random effects to obtain the estimation of nonparametric regression function $f_i(t)$.

In the joint modeling method based on longitudinal data and survival data that is provided according to the embodiment of the present invention, first an expression of a longitudinal index mean function and an expression of a longitudinal index cumulative change rate function are determined; then a longitudinal data sub-model and a survival data sub-model are constructed respectively based on the longitudinal index mean function and the longitudinal index cumulative change rate function; and finally, a covariance matrix structure of a random error in the longitudinal data sub-model is determined based on a calculation model selection criterion, a maximum likelihood estimation value of parameters in the longitudinal data sub-model and the survival data sub-model is calculated by adopting an EM algorithm, and a multidimensional random effect in the longitudinal index mean function is predicted to obtain the estimation of a smooth function in the longitudinal index mean function. With the method of the present invention, the longitudinal index cumulative change rate can be quantitatively and systematically characterized in the survival data model so that the survival data sub-model is more in line with the real situation.

Embodiment 2

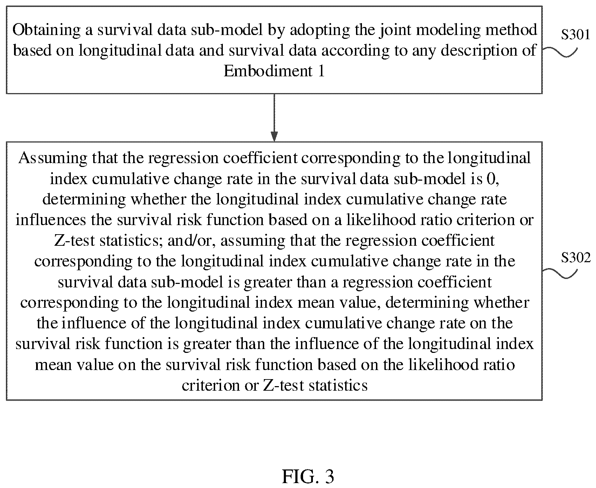

According to this embodiment of the present invention, a method for judging the influence of an index on a model is provided, and as shown in FIG. 3, which is a schematic flowchart diagram of a method for judging the influence of an index on a model provided according to an embodiment of the present invention, the method comprises:

Step S301: obtaining a survival data sub-model by adopting the joint modeling method based on longitudinal data and survival data according to any description of Embodiment 1.

Step S302: assuming that the regression coefficient corresponding to the longitudinal index cumulative change rate in the survival data sub-model is 0, determining whether the longitudinal index cumulative change rate influences the survival risk function based on a likelihood ratio criterion or Z-test statistics; and/or, assuming that the regression coefficient corresponding to the longitudinal index cumulative change rate in the survival data sub-model is greater than a regression coefficient corresponding to the longitudinal index mean value, determining whether the influence of the longitudinal index cumulative change rate on the survival risk function is greater than the influence of the longitudinal index mean value on the survival risk function based on the likelihood ratio criterion or Z-test statistics.

The likelihood ratio criterion is to divide the likelihood function value when the hypothesis holds by the likelihood function value when the parameter is unlimited; and Z-test statistics is to divide the estimated value of the parameter by the standard deviation of the parameter and then take the absolute value.

The embodiment of the present invention provides a method for judging the influence of an index on a model, in which firstly, the longitudinal index cumulative change rate is quantitatively and systematically characterized through a statistical algorithm and a corresponding survival data model is generated, and then based on the likelihood ratio criterion or Z-test statistics, the influence of the longitudinal index cumulative change rate on the survival data model is judged and the influence of the longitudinal index mean value and the longitudinal index cumulative change rate on the survival data model is compared.

Embodiment 3

According to this embodiment of the present invention, a computer-readable storage medium is provided, the types of which may include various media that can store program codes, such as a USB flash disk, a mobile hard disk, a Read-Only Memory (ROM), a Random Access Memory (RAM), a magnetic disk or an optical disk. The computer-readable storage medium stores a computer program which, when being executed by a processor, causes the processor to execute the steps of the joint modeling method based on longitudinal data and survival data described in the first embodiment and the steps of the method of judging the influence of an index on a model described in the second embodiment.

As can be clearly appreciated by those of ordinary skill in the art from the above description of the embodiments, the embodiments may be implemented by software plus a general hardware platform or, of course, by hardware. Based on this understanding, the essential part of the aforesaid technical solutions or the part thereof that contributes to the related art may be implemented in the form of a computer software product. The computer software product may be stored in a computer readable storage medium such as a ROM/ RAM, a magnetic disk, an optical disk or the like, and comprise a plurality of instructions that enable a computer device (which may be a personal computer, a server, or a network device or the like) to execute the method(s) described in the individual embodiments or some portions of the embodiments.

Finally it shall be noted that, the above embodiments are only used to describe but not to limit the technical solutions of the present invention; and within the spirits of the present invention, technical features of the above embodiments or different embodiments may also be combined with each other, the steps may be implemented in an arbitrary order, and many other variations in different aspects of the present invention described above are possible although, for purpose of simplicity, they are not provided in the details. Although the present invention has been detailed with reference to the above embodiments, those of ordinary skill in the art shall appreciate that modifications can still be made to the technical solutions disclosed in the above embodiments or equivalent substations may be made to some of the technical features, and the corresponding technical solutions will not essentially depart from the scope of the embodiments of the present application due to such modifications or substations.

**Claims**

1. A joint modeling method based on longitudinal data and survival data, being **characterized in that**, the method comprising:

   determining an expression of a longitudinal index mean function and an expression of a longitudinal index cumulative change rate function, wherein a longitudinal index mean value in the longitudinal index mean function depends on a first reference independent variable in a parameter form and depends on a smooth function based on a multidimensional random effect in a time form, and a longitudinal index cumulative change rate in the longitudinal index cumulative change rate function is described as a power root of a varicose measure of the longitudinal index mean function;
   constructing a longitudinal data sub-model, wherein longitudinal observation data in the longitudinal data sub-model at least depends on the longitudinal index mean value and a random error;
   constructing a survival data sub-model, wherein a survival risk function in the survival data sub-model at least depends on the longitudinal index mean value and the longitudinal index cumulative change rate;
   determining a covariance matrix structure of the random error based on a calculation model selection criterion; and
   calculating a maximum likelihood estimation value of parameters in the longitudinal data sub-model and the survival data sub-model by adopting an EM algorithm, and predicting the multidimensional random effect to obtain the estimation of the smooth function;

   the expression of the longitudinal index cumulative change rate function is:

   $$I_i(t) = \left[ \int_{t_0}^{t} \{m_i''(s)\}^2 \, ds \right]^{1/2}$$

   or

   $$I_i(t) = \left[ \int_{t_0}^{t} \omega_{\sigma_2}(t - s)\{m_i''(s)\}^2 \, ds \right]^{1/2}$$

   wherein $m_i(s)$ is the longitudinal index mean function of the ith individual at time s, $m_i''(s)$ is the second derivative of $m_i(s)$, $I_i(t)$ represents the longitudinal index cumulative change rate of $m_i(t)$ over a time period [$t_0$, $t$], $\omega_{\sigma_2}(t - s)$ is a weight function about the parameter $\sigma_2$, and the smaller the distance between s and t is, the greater the weight of $\omega_{\sigma_2}(t - s)$ will be;
   the expression of the longitudinal index mean function is:

$$\begin{cases} m_i(t) = x_i^T \beta + f_i(t) \\ f_i(t) = \sum_{k=1}^{q} (\xi_k + b_{ik}) B_k(t) \end{cases}$$

wherein xT is a first reference independent variable vector of the ith individual, $\beta$ is a regression coefficient corresponding to $x_i^T$, $f_i(t)$ is the smooth function of the ith individual, q represents the dimension of the random effect, $\xi_k$ is a fixed effect parameter of a $k$th dimension, $b_{ik}$ represents a random effect parameter of the $k$th dimension of the ith individual, and $B_k(t)$ represents a B-spline basis function corresponding to the $k$th dimension;

the longitudinal data sub-model is:

$$Y_{ij} = m_i(t_{ij}) + w_{ij}^T \eta + \epsilon_{ij}$$

wherein $Y_{ij}$ is an observation value of a random variable $Y$ at a jth time point $t_{ij}$ for the ith individual, $j = 1,2, ..., n_i$, $n_i$ represents the total number of repeated observations of $Y$ before a survival time $T_i$ for the ith individual, $w_{ij}$ is an external independent variable corresponding to the ith individual at the jth time point, $\eta$ is an unknown parameter corresponding to $w_{ij}$, and $\varepsilon_{ij}$ is the random error of the $i$th individual at the jth time point.

the survival data sub-model is:

$$\lambda_i(t) = \lambda_0(t) exp \left( z_i^T \gamma + \alpha_1 m_i(t) + \alpha_2 \left[ \int_{t_0}^{t} \{m_i''(s)\}^2 \, ds \right]^{1/2} \right)$$

or

$$\lambda_i(t) = \lambda_0(t) exp \left\{ z_i^T \gamma + \alpha_1 \int_{t_0}^{t} \omega_{\sigma_1}(t-s) m_i(s) ds + \alpha_2 \left[ \int_{t_0}^{t} \omega_{\sigma_2}(t-s) \{m_i''(s)\}^2 \, ds \right]^{1/2} \right\}$$

wherein $\lambda_0(t)$ is a reference risk function, $z_i^T$ is a second reference independent variable vector of the ith individual, $\gamma$ is a co-parameter corresponding to $z_i^T$, $\alpha_1$ is a co-parameter of the longitudinal index mean value $m_i(t)$ at a current time point t, $\alpha_2$ is a co-parameter of the cumulative change rate of $m_i(t)$ before the current time point t; $\omega_{\sigma_1}(t-s)$ is a weight function about the parameter $\sigma_1$, and $\omega_{\sigma_2}(t-s)$ is a weight function about the parameter $\sigma_2$, so that the smaller the distance between s and $t$ is, the greater the weight of the $\omega_{\sigma_1}(t-s)$ and $\omega_{\sigma_2}(t-s)$ will be;

the longitudinal index include blood pressure index and/or blood glucose index, the first reference independent variable vector and the second reference independent variable vector include an individual's age and gender, and the external independent variable is an external factor variable that affects the blood pressure index or the blood glucose index.

2. The method according to Claim 1, being **characterized in that**, the method comprises:
selecting one of two survival data sub-models as the survival data sub-model for data analysis based on the calculation model selection criterion.

3. The method according to Claim 1, being **characterized in that**, the random error obeys a multivariate normal distribution with a mean value of 0 and a covariance matrix of $\Sigma_i$, and the covariance matrix $\Sigma_i$ is modeled based on the following equation:

$$T_i \Sigma_i T_i^T = D_i$$

wherein $T_i$ is a lower triangular matrix with a diagonal element of 1, while $D_i$ is a diagonal matrix with a positive diagonal element, the lower triangular element of $T_i$ is $-\phi_{ijk}(1 \leq k < j \leq n_i)$, the diagonal element of $D_i$ is $\sigma^2_{ij}(1 \leq j \leq n_i)$, $\phi_{ijk}$ is an autoregressive coefficient of $Y_{ij}$ to $Y_{ik}(k = 1,2, ...j - 1)$, and $\sigma^2_{ij}$ is an innovation variance.

4. The method according to Claim 3, being **characterized in that**, the method further comprises:

modeling the $\phi_{ijk}$ and the $\sigma^2_{ij}$ based on the following equation:

$$\begin{cases} \phi_{ijk} = a^T_{ijk}\tau \\ log(\sigma^2_{ij}) = c^T_{ij}\zeta \end{cases}$$

wherein $a_{ijk}$ and $c_{ij}$ are known independent variables, and $\tau$ and $\zeta$ are model parameters.

5. A method for judging the influence of an index on a model, being **characterized in that**, the method comprising:

obtaining a survival data sub-model by adopting the joint modeling method based on longitudinal data and survival data according to any of Claims 1 to 4;
assuming that the regression coefficient corresponding to the longitudinal index cumulative change rate in the survival data sub-model is 0, determining whether the longitudinal index cumulative change rate influences the survival risk function based on a likelihood ratio criterion or Z-test statistics; and/or,
assuming that the regression coefficient corresponding to the longitudinal index cumulative change rate in the survival data sub-model is greater than a regression coefficient corresponding to the longitudinal index mean value, determining whether the influence of the longitudinal index cumulative change rate on the survival risk function is greater than the influence of the longitudinal index mean value on the survival risk function based on the likelihood ratio criterion or Z-test statistics.

6. A computer-readable storage medium, being **characterized in that**, the computer-readable storage medium storing a computer program which, when being executed by a processor, causes the processor to execute the steps of the method according to any of Claims 1 to 5.

```
┌──────────────────────────────────────────────────────────────────────┐
│ Determining an expression of a longitudinal index mean function and an │  S101
│ expression of a longitudinal index cumulative change rate function     │
└──────────────────────────────────────────────────────────────────────┘
                              │
                              ▼
┌──────────────────────────────────────────────────────────────────────┐
│ Constructing a longitudinal data sub-model, wherein longitudinal       │  S102
│ observation data in the longitudinal data sub-model at least depends   │
│ on the longitudinal index mean value and a random error                │
└──────────────────────────────────────────────────────────────────────┘
                              │
                              ▼
┌──────────────────────────────────────────────────────────────────────┐
│ Constructing a survival data sub-model, wherein a survival risk        │  S103
│ function in the survival data sub-model at least depends on the        │
│ longitudinal index mean value and the longitudinal index cumulative    │
│ change rate                                                            │
└──────────────────────────────────────────────────────────────────────┘
                              │
                              ▼
┌──────────────────────────────────────────────────────────────────────┐
│ Determining a covariance matrix structure of the random error based    │  S104
│ on a calculation model selection criterion                             │
└──────────────────────────────────────────────────────────────────────┘
                              │
                              ▼
┌──────────────────────────────────────────────────────────────────────┐
│ Calculating a maximum likelihood estimation value of parameters in the │  S105
│ longitudinal data sub-model and the survival data sub-model by         │
│ adopting an EM algorithm, and predicting the multidimensional random   │
│ effect to obtain the estimation of the smooth function                 │
└──────────────────────────────────────────────────────────────────────┘
```

FIG. 1

FIG. 2

Obtaining a survival data sub-model by adopting the joint modeling method based on longitudinal data and survival data according to any description of Embodiment 1 — S301

Assuming that the regression coefficient corresponding to the longitudinal index cumulative change rate in the survival data sub-model is 0, determining whether the longitudinal index cumulative change rate influences the survival risk function based on a likelihood ratio criterion or Z-test statistics; and/or, assuming that the regression coefficient corresponding to the longitudinal index cumulative change rate in the survival data sub-model is greater than a regression coefficient corresponding to the longitudinal index mean value, determining whether the influence of the longitudinal index cumulative change rate on the survival risk function is greater than the influence of the longitudinal index mean value on the survival risk function based on the likelihood ratio criterion or Z-test statistics — S302

FIG. 3

**EP 4 597 516 A1**

| Europäisches Patentamt European Patent Office Office européen des brevets | **EUROPEAN SEARCH REPORT** | Application Number<br>EP 24 21 6826 |
|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DATABASE BIOSIS [Online]<br>BIOSCIENCES INFORMATION SERVICE,<br>PHILADELPHIA, PA, US;<br>25 May 2023 (2023-05-25),<br>WANG CHUNYU ET AL: "Modeling biomarker variability in joint analysis of longitudinal and time-to-event data",<br>XP002813315,<br>Database accession no. PREV202400783132<br>* abstract *<br>& BIOSTATISTICS,<br>vol. 25, no. 2, 25 May 2023 (2023-05-25),<br>pages 577-596,<br>ISSN: 1465-4644(print), DOI:<br>10.1093/BIOSTATISTICS/KXAD009<br>- - - - - | 1-6 | INV.<br>G16H50/30<br>G16H50/70 |
| A | Samuel Louis George Brilleman: "Joint longitudinal and time-to-event models: development, implementation and applications in health research",<br>,<br>22 March 2019 (2019-03-22), XP093156829,<br>DOI: 10.4225/03/5b35b428cd48a<br>Retrieved from the Internet:<br>URL:https://www.sambrilleman.com/files/brilleman_thesis.pdf<br>* abstract *<br>* Chapter 2.5.3, Chapter 4.2 *<br>- - - - - | 1-6 | |

**TECHNICAL FIELDS SEARCHED    (IPC)**

G16H

The present search report has been drawn up for all claims

| Place of search | Date of completion of the search | Examiner |
|---|---|---|
| Munich | 12 May 2025 | Samulowitz, Michael |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)